# EUROPÄISCHE PATENTANMELDUNG

(11) **EP 1 064 961 A1**
(43) Veröffentlichungstag der Anmeldung: **03.01.2001**
(21) Anmeldenummer: 00109144.6
(22) Anmeldetag: 05.05.2000
(51) Int. Cl.: A61M 5/31, A61J 1/00

(54) **Spritze für medizinische Zwecke**

(30) Priorität: 15.06.1999 DE 19927201
(71) Anmelder: Schott Glas, 55122 Mainz (DE); Carl Zeiss Stiftung Trading as Schott Glaswerke, 55122 Mainz (DE)
(72) Erfinder: Heinz, Jochen, Dr., 55578 Vendersheim (DE); Spallek, Michael, Dr., 55218 Ingelheim (DE)
(74) Vertreter: Fuchs Mehler Weiss & Fritzsche

(57) **Zusammenfassung**

Derartige Spritzen, die vorzugsweise vorgefüllt sind, besitzen einen Kunststoff-Spritzenkorpus (1), der bei einer speziellen Anwendungsform zum Transferieren von medizinischen Substanzen aus Medizin-/Injektionsfläschchen (10), die durch einen elastomeren Stopfen (11) verschlossen sind, in einen Infusionsbehälter ausgebildet ist.

Um die beim Umgang mit den Spritzen durch die typischerweise zum Durchstechen verwendeten medizinischen Kanülen (Injektionsnadeln) bedingten Gefahren zu vermeiden, ist gemäß der Erfindung der Spritzenkorpus (1) frontseitig mit einem Hohldorn (9) aus Kunststoff zum Durchstechen der elastomeren Stopfen (11) versehen.

Vorzugsweise ist der Hohldorn (9) einstückig am Spritzenkorpus (1) angeformt.

## Beschreibung

Die Erfindung bezieht sich auf eine Spritze für medizinische Zwecke mit einem Kunststoff-Spritzenkorpus, ausgebildet zum Transferieren von medizinischen Substanzen aus Behältnissen, die durch einen elastomeren Verschluß verschlossen sind, in einen Infusionsbehälter.

Sie bezieht sich dabei vorzugsweise auf vorgefüllte Spritzen. Dabei handelt es sich um vorfüllbare Spritzen aus Glas oder Kunststoff, die an ihrem Kopfende entweder eine eingeklebte Metallkanüle oder einen Konus zur Aufnahme von medizinischen Kanülen aufweisen.

Alle vorgenannten Spritzen weisen den gravierenden Nachteil auf, daß für den Anwender stets die Gefahr besteht, sich an der Kanüle zu verletzen. Deshalb wurden verschiedenste, aufwendige Systeme von Sicherheitsspritzen entwickelt, welche die Verletzungsgefahr durch die Kanüle vermindern sollen.

Die bekannten vorgefüllten Spritzen sind zu einer direkten Injektion beim Patienten gut geeignet. Zur Aufnahme eines flüssigen Medikaments aus einem mit einem Elastomerverschluß versehenen, normierten Medikamentenfläschchen oder gar eines pulverförmigen Medikaments aus einem Standardinjektionsfläschchen sind die genannten Spritzen wenig geeignet.

Präparate, wie sie vorwiegend in der Medizin, der Pharmazeutik und Diagnostik und der Labor- bzw. Analysetechnik verwendet werden, kommen nämlich üblicherweise in vorgenannten Behältnissen in den Handel bzw. werden in dieser Form an den Endverbraucher (Anwender) geliefert.

Die Präparate müssen im Anwendungsfall den verschlossenen Behältnissen in geeigneter Weise entnommen werden, möglichst ohne ihre Funktion zu beeinträchtigen. Häufig müssen dabei diese Präparate noch vor ihrer Anwendung mit anderen Substanzen versetzt werden. Beispielsweise ist dies der Fall, wenn die Präparate in fester Form vorliegen, d. h,. ein Pulver oder ein Lyophilisat sind, die vor ihrer Anwendung in eine Lösung überführt werden müssen. Ein anderes Beispiel ist die Verdünnung eines flüssigen, konzentrierten Präparates.

Der Zugang zu dem Behälterinneren erfolgt typischerweise unter Durchstechung des Elastomerverschlusses mittels Kanüle und Einmalspritze als "Zwischenträger". Soll z. B. ein flüssiges Präparat appliziert werden, kann nach Durchstechen des Verschlußstopfens des Behältnisses die Spritze aufgezogen und anschließend appliziert werden. Soll vorher eine Mischung mit einer anderen flüssigen Substanz stattfinden, wird zunächst die Spritze mit dieser Substanz aufgezogen, sowie dann der Stopfen des Behältnisses unter Entleerung der Spritze durchstochen. Nach Mischen der Substanzen in dem Behältnis wird danach die Spritze mit der Mischlösung aufgezogen und anschließend appliziert.

Entsprechend sind die Verfahrensschritte, wenn die Wirksubstanz in fester Form vorliegt. Zunächst wird die Spritze mit dem Lösungsmittel aufgezogen und danach unter Durchstechung des Stopfens des Behältnisses in dieses entleert, wobei ggf. noch ein Mischvorgang mit einer anderen Substanz durchgeführt werden kann. Danach wird die Spritze mit der zu applizierenden Endlösung aufgezogen.

Ist die Spritze eine Spritze der vorbeschriebenen Art mit einer medizinischen Kanüle, so kann es bei dem Durchstechen des Elastomerverschlusses eines Fläschchens leicht zur Beschädigung der verwendeten medizinischen Kanüle kommen, was ein Auswechseln der Kanüle routinemäßig notwendig macht. Mit dem Auswechseln in der Kanüle sind jedoch wiederum Verletzungsgefahren des medizinischen Personals oder des Anwenders selbst verbunden. Ferner kommt es durch die Verwendung von medizinischen Kanülen zum Durchstechen von elastomeren Verschlüssen zum Ausstanzen von Elastomer-Partikeln und somit zu der großen Gefahr, daß diese Partikel zusammen mit dem Medikament verabreicht werden.

Es ist bekannt, diese Nachteile durch geschlossene Behältnisse mit einem integrierten "transfer set" zu vermeiden. Unter einem "transfer set" soll in diesem Zusammenhang eine Zusatzeinrichtung zum Überführen, d. h. "Transferieren" des Präparates, direkt oder nach Mischung mit einer anderen Substanz, in einen für den Gebrauch, die Applikation, vorgesehenen Endbehälter oder direkt in einen Applikator, verstanden werden.

Diese Transfer-Aufsätze eignen sich insbesondere zum Auflösen von pulverförmigen Medikamenten in dem jeweiligen Medikamenten-/Injektionsfläschchen. Sie sind jedoch nicht dafür geeignet, pulverförmige Medikamente direkt in eine starre Infusionsflasche zu überführen, da der notwendige Überführungsdruck nicht aufgebracht werden kann.

Der Erfindung liegt die Aufgabe zugrunde, die eingangs bezeichnete Spritze für medizinische Zwecke mit einem Kunststoff-Spritzenkorpus, ausgebildet zum Transferieren von medizinischen Substanzen aus Behältnissen, die durch einen elastomeren Verschluß verschlossen sind, in einen Infusionsbehälter, so auszubilden, daß eine sichere Überführung eines in dem separaten Behältnis befindlichen pulverförmigen Präparates sowohl in eine formfeste Infusionsflasche als auch in einen nicht formfesten Infusionsbeutel ohne die Verwendung von medizinischen Injektions-Kanülen möglichst einfach gewährleistet ist.

Die Lösung dieser Aufgabe gelingt gemäß der Erfindung dadurch, daß der Spritzenkorpus frontseitig Injektionskanülen-frei und mit einem Hohldorn aus Kunststoff zum Durchstechen des elastomeren Verschlusses versehen ist.

Durch die erfindungsgemäßen Maßnahmen wird eine Spritze für medizinische Zwecke, insbesondere eine vorgefüllte Spritze geschaffen, die ohne Verwendung von medizinischen Injektions-Kanülen zum Durchstechen von elastomeren Verschlüssen verwendet werden kann.

Der Hohldorn, der auch als Spike bezeichnet wird, ist dabei ausreichend lang und scharf ausgebildet für das Durchstechen von normgerechten elastomeren Verschlüssen von Infusionsflaschen aus Kunststoff bzw. aus Glas und Infusionsbeuteln, insbesondere normierte Standardinjektionsstopfen, bzw. von normierten Gefriertrocknungsstopfen bzw. von normierten Gefriertrocknungsstopfen für Infusionsflaschen.

Der Hohldorn stellt sicher, daß die Gefahr der Verletzung und der Injektion von Elastomer-Partikeln für die Anwender wesentlich geringer als im Fall von medizinischen Kanülen ist.

Der Hohldorn durchdringt dabei nicht nur auf einfache Weise den Verschluß der separaten Behältnisse, insbesondere von Medizin-/Injektionsfläschchen, sondern auch die Verschlüsse von Injektionsflaschen bzw. Infusionsbeuteln.

Die DE 77 02 734 U1 beschreibt zwar eine Anordnung zum Transferieren von medizinischen Substanzen aus oder in einen Behälter, der durch einen elastomeren Stopfen verschlossen ist, mittels einer Spritze, jedoch geschieht dieses Transferieren mit unterschiedlichen Mitteln. Während im Fall der Erfindung der Spritzenkorpus frontseitig direkt mit einem Kunststoff-Hohldorn als Teil der Spritze versehen ist, mittels dem bei jedem Transfervorgang der elastomere Stopfen durchstochen wird, ist im bekannten Fall getrennt von der Spritze, eine Entnahme- und Belüftungskanüle mit einem frontseitigen hohlen Einstechdorn nach Durchstechen des Stopfens dauerhaft an dem Behälter für einen Mehrfachgebrauch installiert. Diese Kanüle besitzt einen Filter zum Belüften des Behälters mit keimfreier Luft sowie einen gummielastischen Einsatz zum Verschließen der Bohrung der Kanüle bei Nichtgebrauch. Die Spritze selbst dagegen besitzt im bekannten Fall lediglich einen üblichen konusartigen Spritzenkopf, mit dem die Enden des geschlitzten gummielastischen Einsatzes im Transferfall spreizbar sind, um die Öffnung für den Flüssigkeitstransfer freizugeben.

Die DE 77 02 734 U1 zeigt daher nicht die beanspruchte, erfindungsgemäße Spritze, sondern einen speziellen, mittels eines Einstechdornes in den Stopfen eines Behälters mit medizinischer Flüssigkeit eingeführten Verschluß-Einsatzes, der es erlaubt, mit konventionellen Spritzen, ohne weiteres Durchstechen des Stopfens, einen wiederholten Transfer der medizinischen Flüssigkeit durchzuführen.

Die EP 0 306 606 zeigt eine Transfer-Spritze mit zwei Kanülen, von denen eine dem Durchstechen des Stopfens eines Behälters und der Belüftung beim Entnehmen der medizinischen Flüssigkeit aus dem Behälter, und die andere der "eigentlichen" Injektion für das Verabreichen der medizinischen Flüssigkeit am Patienten dient.

In beiden Fällen liegen jedoch typische Injektions-Kanülen vor, die aus einem Metall gebildet sind, d.h. die bekannte "Aspirationskanüle 01" ist kein Einstech-Hohldorn wie im Fall der Erfindung, und auch nicht aus Kunststoff.

Im Fall der Erfindung geht es jedoch gerade darum, die Verwendung von medizinischen Kanülen zu vermeiden.

Durch die DE 21 64 363 A1 ist eine konventionelle Injektionsspritze bekannt geworden, mit einer Injektionsnadel aus Kunststoff, die kein Einstech-Hohldorn im Sinne der Erfindung ist, wie auch die bekannte Spritze generell keine Transfer-Spritze im Sinne der Erfindung ist.

Die Spritze kann gemäß einer Weiterbildung der Erfindung so ausgebildet sein, daß der Kunststoff-Hohldorn einen einstückigen Halter zum Aufstecken auf einen konusartig geformten Spritzenkopf des Spritzenkorpus aufweist.

In diesem Fall ist die Spritze insoweit zweistückig ausgeführt. Spritzenkorpus und Hohldorn werden als separate Teile gefertigt. Der Hohldorn wird dann mit einem Halter unlösbar auf dem Spritzenkopf aufgesetzt.

Gemäß einer anderen Weiterbildung der Erfindung wird jedoch vorzugsweise die Spritze einstückig ausgebildet, indem das Austrittsende des Spritzenkorpus unmittelbar als Hohldorn ausgeformt ist.

Der Hohldorn kann auf verschiedene Weise ausgebildet werden je nach Anwendungszweck, d. h., welchen Typ eines Verschlusses er durchdringen soll. So ist es gemäß einer ersten Ausgestaltung denkbar, daß der Hohldorn hohlzylindrisch ausgebildet ist, d. h. über seine Länge einen konstanten Durchmesser aufweist.

Gemäß einer anderen Ausgestaltung kann der Hohldorn konisch verjüngt ausgebildet sein, was die Festigkeit des Hohldornes an seiner Basis verstärkt.

Auch das Einstichende des Hohldornes kann verschiedenartig ausgebildet sein. Gemäß einer ersten Ausgestaltung weist der Hohldorn am Einstichende eine, sich über den gesamten Durchmesser erstreckende Schräge auf, wodurch eine exzentrische Spitze gegeben ist.

Gemäß einer anderen Ausgestaltung der Erfindung ist der Hohldorn zum Einstichende hin kegelförmig spitz zulaufend ausgebildet, so daß das Einstichende mittig liegt. Dabei kann der Hohldorn nur einen einzigen exzentrisch angeordneten Strömungskanal aufweisen oder er kann alternativ dazu zwei symmetrisch angeordnete Strömungskanäle besitzen. Im letzteren Fall läßt sich ein größerer Durchfluß erzielen.

Um eine sterile Belüftung des Applikationsbehältnisses zu gewährleisten, ist gemäß einer Ausgestaltung der Erfindung am korpusseitigen Ende des Hohldornes ein Stutzen zum Aufstecken eines Sterilfilters ausgebildet.

Weitere ausgestaltende Merkmale und Vorteil der Erfindung ergeben sich anhand der Beschreibung von in den Zeichnungen dargestellten Ausführungsbeispielen.

Es zeigen:
- Fig. 1: in einer Längsschnitt-Darstellung die Grundform einer konventionellen Spritze im Lagerzustand, die die Basis für die erfindungsgemäße Spritze bildet,
- Fig. 2: die applizierbereit montierte Spritze nach Fig. 1 mit einem aufgesetzten, zylindrischen Hohldorn,
- Fig. 3: die Spritze nach Fig. 2 im Applikationszustand mit durchstochenen Stopfen eines Medikamentenfläschchens,
- Fig. 4: in einer Längsschnitt-Darstellung eine Spritze nach Fig. 2, jedoch mit konisch zulaufendem Hohldorn,
- Fig. 5: die Spritze nach Fig. 4 im Applikationszustand analog Fig. 3,
- Fig. 6: in einer Längsschnitt-Darstellung eine weitere Ausführungsform der Spritze mit einem einstückig an dem Spritzenkorpus angeformten Hohldorn im Lagerzustand,
- Fig. 7: die Spritze nach Fig. 6 im Applikationszustand analog Fig. 3,
- Fig. 8: in einer Längsschnitt-Darstellung eine Spritze entsprechend Fig. 6, die zusätzlich mit einem Sterilfilter versehen ist,
- Fig. 9: die Spritze nach Fig. 8 im Applikationszustand,
- Fig. 10: in einer Längsschnitt-Darstellung eine weitere Ausführungsform mit einstückig angeformtem Hohldorn, der kegelförmig spitz laufend ausgebildet ist und zwei symmetrisch angeordnete Strömungskanäle aufweist,
- Fig. 11: die Spritze nach Fig. 10 im Applikationszustand,
- Fig. 12: in einer Längsschnitt-Darstellung eine Spritzenausführung entsprechend Fig. 10, jedoch mit nur einem asymmetrischen Strömungskanal,
- Fig. 13: eine Halterung einer Spritze nach Fig. 6 während der Applikation in ein Medikamentenfläschchen, und
- Fig. 14: eine Spritze nach Fig. 6 mit auf dem Hohldorn aufgeschobener Standardkanüle.

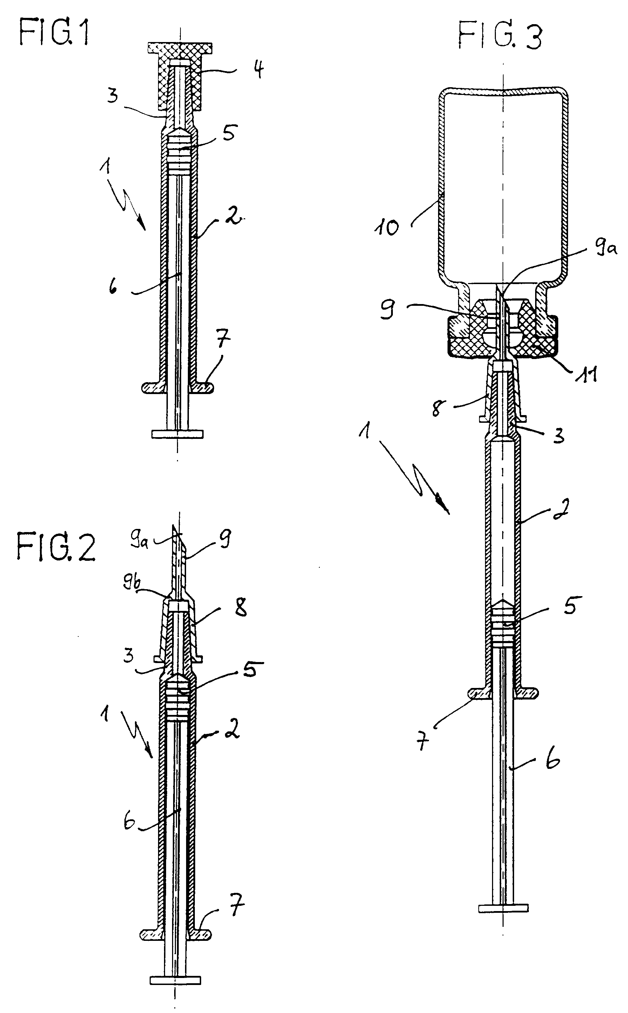

Die Fig. 1 zeigt eine konventionelle Kunststoffspritze mit einem Spritzenkorpus 1, der aus einem Spritzenzylinder 2 und einem daran angeformten konischen Spritzenkopf 3 besteht, der durch eine Elastomerkappe, ein sog. Tip-Cap 4, verschließbar ist. Ein Kolbenstopfen 5 mit einer Kolbenstange 6 und eine Fingerleiste 7 am Spritzenzylinder 2 ergänzen den Spritzenkorpus 1 zu der konventionellen Spritze.

Die Fig. 1 zeigt die Spritze in ihrer Grundform. Ist die Spritze vorgefüllt und mit der Elastomerkappe verschlossen, sitzt der Kolbenstopfen 5 am unteren Ende des Spritzenzylinders 2 ohne angebrachte Kolbenstange 6.

Die Fig. 2 zeigt die erfindungsgemäß ausgebildete Spritze nach Fig. 1 im applizierbereiten Zustand. Dazu wird auf den konischen Spritzenkopf 3 nach Abnahme der Elastomerkappe 4 mittels eines komplementär geformten Halters 8 ein einstückig damit ausgeformter Hohldorn 9, der Spike, aufgesetzt. Der Halter 8 mit Hohldorn 9 bestehen dabei ebenfalls aus Kunststoff und sind vorzugsweise, wie der Spritzenkorpus, Spritzgießteile.

Der Hohldorn 9 besitzt am frontseitigen Ende seines inneren Strömungskanals eine scharfkantige Abschrägung 9a, die ein Einstechen in einen Elastomerstopfen erleichtert, ohne daß eine beachtliche Verletzungsgefahr besteht.

Die Fig. 3 zeigt die erfindungsgemäße Spritze beim Applizieren in ein Medizinfläschchen 10. Der Hohldorn 9 durchdringt den Stopfen 11 dieses Medizinfläschchens 10, so daß, je nach Fall, der Inhalt der Spritze in das Medizinfläschchen abgegeben werden kann, z. B. zum Verdünnen einer darin enthaltenen Lösung oder zum Auflösen einer festen, z.B. gefriergetrockneten, medizinischen Substanz. Danach kann die Spritze wieder aufgezogen werden, um deren Inhalt anschließend in ein Infusionsbehältnis zu transferieren. Dieses Infusionsbehältnis kann eine Infusionsflasche aus Glas oder Kunststoff mit einem genormten Verschluß oder ein Infusionsbeutel mit einer üblichen, durchstechbaren Zugangsöffnung, auch Port genannt, sein.

Bei der Spritze nach den Figuren 1 bis 3 besitzt der Halter 8 des Hohldornes 9 am frontseitigen Ende eine kegelstumpfartige Verjüngung 9b, an die sich der Hohldorn 9 anschließt, der über seine gesamte Länge durchmessergleich ist.

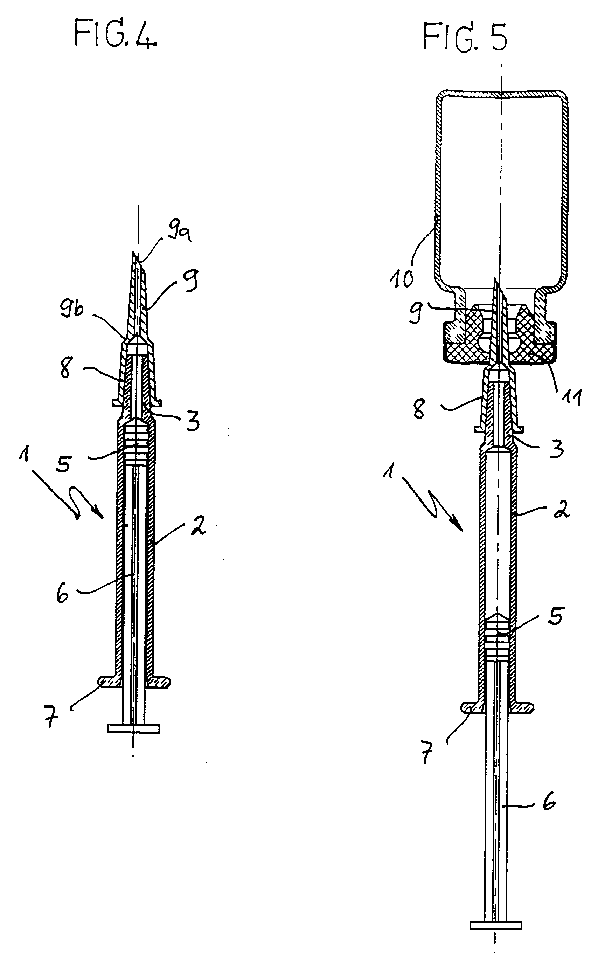

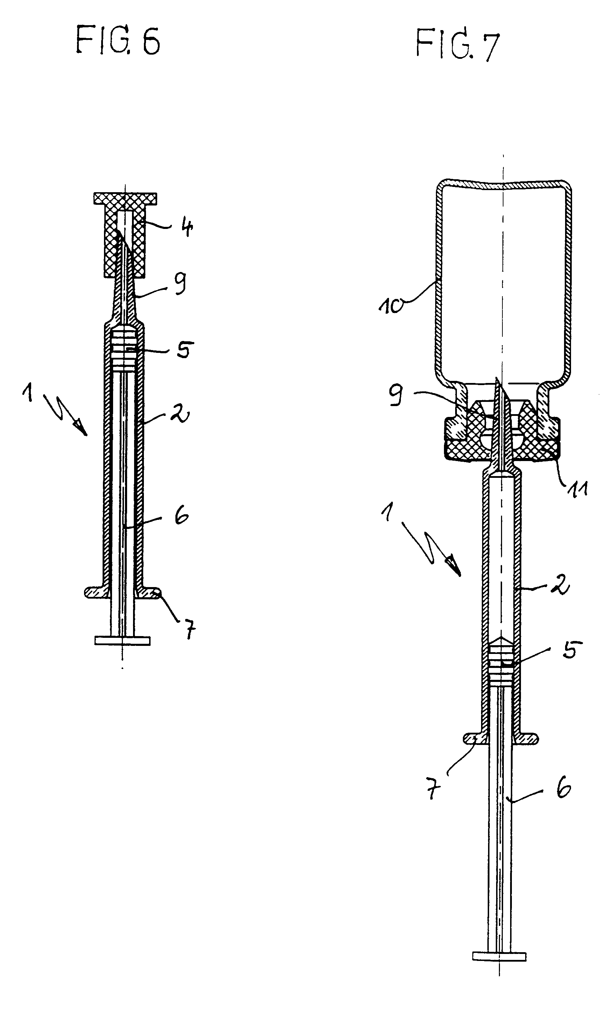

Bei der Spritze nach den Fig. 4 und 5 ist die kegelstumpfartige Verjüngung 9b weniger stark ausgeprägt als im Fall der Figuren 1 bis 3, und der Hohldorn 9 verjüngt sich konusförmig zu seiner Spitze hin. Ferner ist der Hohldorn länger als im ersten Beispiel. Er kann dadurch, wie aus der Fig. 5 ersichtlich ist, tiefer in das Behältnis 10 eindringen.

Im übrigen entspricht die Ausführung der Spritze nach den Figuren 4 und 5 derjenigen nach den Figuren 1 bis 3.

Bei der zweistückigen Ausführung der Spritze nach den Figuren 1 bis 5 ist der Hohldorn 9 mit seinem Halter 8 ein separates Teil, das auf den Spritzenkopf 3 aufgesetzt wird.

Die Figuren 6 und 7 zeigen eine Spritze mit einem frontseitigen Ende, das unmittelbar und einstückig als Hohldorn 9 ausgeformt ist, oder anders ausgedrückt, der Spritzenkopf 8 bei der Spritzenausführung nach den Figuren ist bei den Ausführungen nach den Figuren 6 und 7 zugleich der Hohldorn 9.

Die Ausbildung des Hohldornes 9 kann durchmessergleich wie im Fall der Figuren 1 bis 3 sein. Er kann auch, wie dargestellt, eine konisch verjüngte Konfiguration wie im Fall der Figuren 4 und 5 haben.

Die Ausführung nach den Figuren 6 und 7 ist eine bevorzugte Ausführung, weil die gesamte Spritze in einem Arbeitsgang hergestellt werden kann. Außerdem wird durch den Wegfall des Aufsetzens eines Hohldornhalters die Handhabung beim Applizieren einfacher, einhergehend mit einer Verringerung der Verletzungsgefahr.

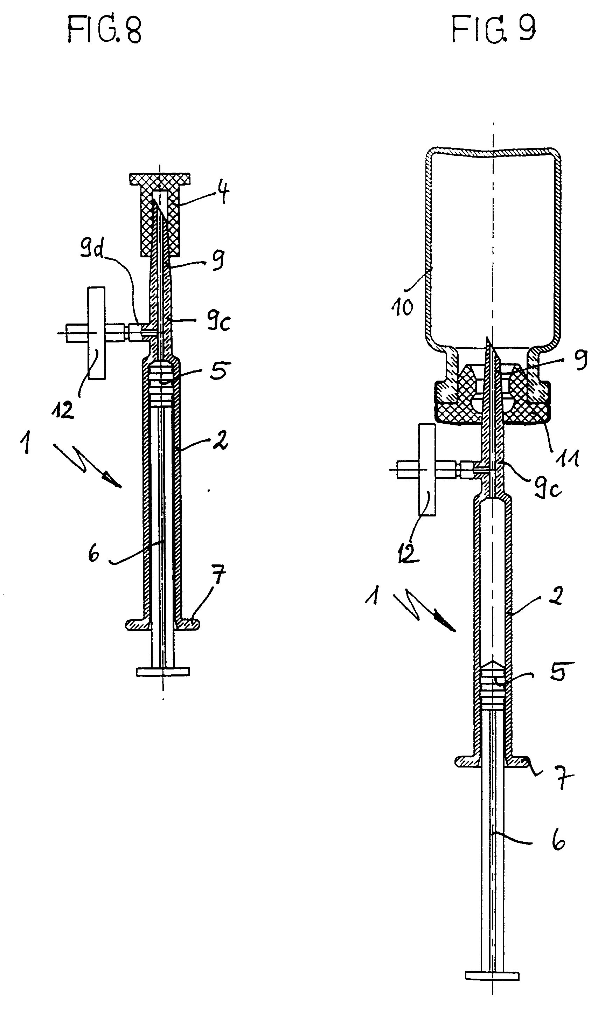

Die Figuren 8 und 9 zeigen eine Spritze analog der einstückigen Ausführung nach den Figuren 6 und 7, wobei die Fig. 8 die Lagerform und Fig. 9 den Verwendungszustand darstellt.

Die Spritzen nach den Figuren 8 und 9 weisen zusätzlich einen Sterilfilter 12 auf, der ein steriles Belüften des Behältnisses 10 beim Applizieren erlaubt. Wenn Flüssigkeit in dieses Behältnis appliziert wird, muß die verdrängte Luft entweichen können, bzw., wenn Flüssigkeit aus dem Behältnis 10 abgezogen wird, muß Luft nachströmen können, die dann steril sein soll.

Zum Anschluß des Sterilfilters 12 weist die Spritze in einem verlängerten hohlzylindrischen Abschnitt 9c des Hohldornes 9, sozusagen im Spritzenkopf, einen angeformten Stutzen 9d auf, der in Strömungsverbindung mit dem inneren Strömungskanal des Hohldornes 9 steht und auf den der Sterilfilter 12 aufgesteckt ist.

Der Sterilfilter kann auch unlösbar am Hohldorn integriert sein.

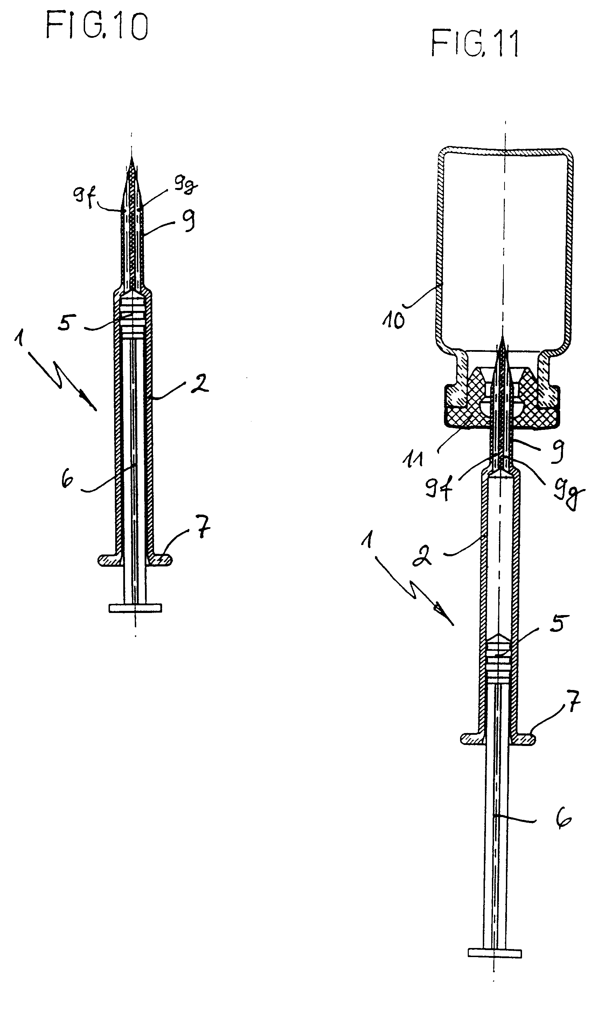

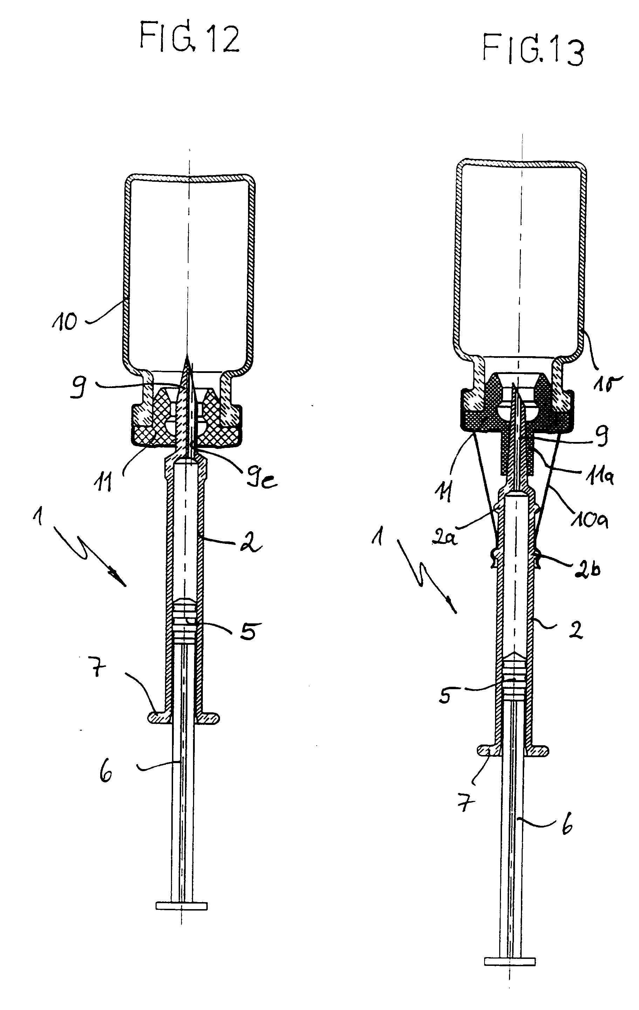

Während bei den bisher beschriebenen Ausführungsformen der Spritze der Hohldorn 9 frontseitig eine durchgehende Schräge aufweist, ist bei den Spritzenausführungen nach den Figuren 10 bis 12 der Hohldorn am Einstichende kegelförmig spitz zulaufend ausgebildet mit seitlichen Öffnungen im Kegelmantel. Bei der Ausführung nach Fig. 12 ist dabei nur ein einziger exzentrischer Strömungskanal 9e vorgesehen, während bei der Ausführung des Hohldornes 9 nach den Figuren 10 und 11 zwei Strömungskanäle 9f, 9g symmetrisch zur Spritzenlängsachse ausgebildet sind.

Die spitz zulaufende Ausbildung des Hohldornes 9, auch als Lanzette bezeichnet, eignet sich bei bestimmten Behältnissen besser zum Einstechen in den Verschluß als die abgeschrägte Ausführung nach den Figuren 1 bis 9 mit asymmetrischer Spitze.

Die Fig. 13 zeigt ein Behältnis 10 mit einem speziellen Verschluß 11 mit angeformten Stutzen 11a und mit einer Halterung 10a für eine Spritze in der Ausführung nach Fig. 6, die wulstartige Ansätze 2a und 2b für einen Formschluß mit komplementär ausgebildeten Enden der Halterungen 10a aufweist.

Diese spezielle Ausführungsform nach Fig. 13 ermöglicht eine Lagerung der Spritze 1 zusammen mit dem Präperatebehältnis 10. In der Lagerform ist der kegelförmige Trichter 10 a in den wulstartigen Ansätzen 2 a (nahe dem kopfseitigen Ende der Spritze) fixiert. In dieser Position wird der Elastomer-Verschluß 11 des Behältnisses nicht durchstochen, vielmehr findet lediglich eine Abdichtung des Hohldornes 9 in dem angeformten Stutzen 11 a statt. In dieser vormontierten Form sind somit Spritze 1 als auch Medikamentenbehältnis 10 sicher verschlossen. Erst kurz vor der Anwendung wird die Spritze in die in Fig. 13 gezeigte Position vorgeschoben, so daß die Halterung 10 a in die wulstartigen Ansätze 2 b eingreift. In dieser Position kann nun der Inhalt der Spritze in den Behälter überführt werden. Nach dem Aufziehen des vermischten Inhalts des Fläschchens in die Spritze wird diese aus dem Stutzen 11 a und der Halterung 10 a herausgezogen und kann nun, wie bereits beschrieben, weiterverwendet werden. Die damit erreichte Vorpositionierung der Spritze auf dem Fläschchen ermöglicht es auch ungeübten Anwendern damit umzugehen.

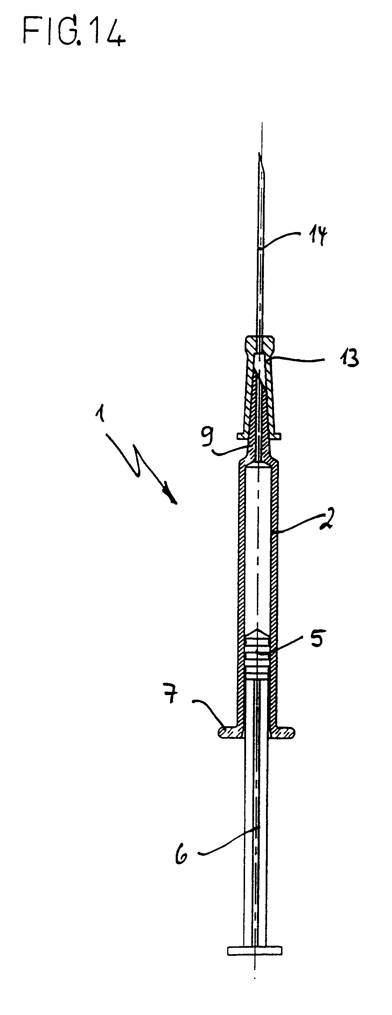

In der Fig. 14 ist eine Spritze entsprechend Fig. 6 dargestellt, auf deren Hohldorn 9 mittels eines Nadelträgers 13 eine Standardkanüle 14 aufschiebbar ist, was eine universelle Verwendbarkeit der Spritze gewährleistet.

Typischerweise ist der Querschnitt des Hohldornes rund. Er kann jedoch auch oval geformt sein.

In den Figuren 1 bis 14 ist der Hohldorn 9 zentrisch am Spritzenkorpus 1 angebracht. Es ist auch denkbar, den Hohldorn azentrisch anzubringen.

Die Spritzen nach den Figuren 1 bis 14 können auch als Fertigspritze ausgebildet sein. Sie sind dann befüllt, beispielsweise mit einem Lösungsmittel.

Die Herstellung der Spritze nach den Figuren 1 bis 14, die Wahl des Kunststoffmaterials, optional die Aufbringung von Sperr- bzw. Gleitschichten und die Befüllung der Spritzen erfolgt gemäß dem Stand der Technik.

## Patentansprüche

1. Spritze für medizinische Zwecke mit einem Kunststoff-Spritzenkorpus (1), ausgebildet zum Transferieren von medizinischen Substanzen aus Behältnissen (10), die durch einen elastomeren Verschluß (11) verschlossen sind, in einen Infusionsbehälter, dadurch gekennzeichnet, daß der Spritzenkorpus (1) frontseitig Injektionskanülen-frei und mit einem Hohldorn (9) aus Kunststoff zum Durchstechen des elastomeren Verschlusses (11) versehen ist.

2. Spritze nach Anspruch 1, dadurch gekennzeichnet, daß der Kunststoff-Hohldorn (9) einen einstückigen Halter (8) zum Aufstecken auf einen konusartig geformten Spritzenkopf (8) des Spritzenkorpus (1) aufweist.

3. Spritze nach Anspruch 1, dadurch gekennzeichnet, daß das Austrittsende des Spritzenkorpus (1) unmittelbar als Hohldorn (9) ausgeformt ist.

4. Spritze nach einem der Ansprüche 1 bis 3, dadurch gekennzeichnet, daß der Hohldorn (9) hohlzylindrisch ausgebildet ist.

5. Spritze nach einem der Ansprüche 1 bis 3, dadurch gekennzeichnet, daß der Hohldorn (9) konisch verjüngt ausgebildet ist.

6. Spritze nach einem der Ansprüche 1 bis 5, dadurch gekennzeichnet, daß der Hohldorn (9) am Einstichende eine, sich über den gesamten Durchmesser erstreckende Schräge (9a) aufweist.

7. Spritze nach einem der Ansprüche 1 bis 5, dadurch gekennzeichnet, daß der Hohldorn (9) zum Einstichende hin kegelförmig spitz zulaufend ausgebildet ist.

8. Spritze nach Anspruch 7, dadurch gekennzeichnet, daß der Hohldorn (9) einen exzentrisch angeordneten Strömungskanal (9e) aufweist.

9. Spritze nach Anspruch 7, dadurch gekennzeichnet, daß der Hohldorn (9) zwei symmetrisch angeordnete Strömungskanäle (9f,g) aufweist.

10. Spritze nach einem der Ansprüche 1 bis 9, dadurch gekennzeichnet, daß am korpusseitigen Ende (9c) des Hohldornes (9) ein Sterilfilter (12) angebracht ist.

11. Spritze nach Anspruch 10, **dadurch gekennzeichnet,** daß ein Stutzen (9 d) zum Aufstecken des Sterilfilters (12) vorgesehen ist.

12. Spritze nach einem der Ansprüche 1 bis 11, **dadurch gekennzeichnet,** daß der Querschnitt des Hohldornes (9) oval geformt ist.

13. Spritze nach einem der Ansprüche 1 bis 12, **dadurch gekennzeichnet,** daß der Hohldorn (9) azentrisch am Spritzenkorpus (1) angebracht ist.

14. Spritze nach einem der Ansprüche 1 bis 13, **dadurch gekennzeichnet,** daß der Hohldorn (9) so geformt ist, daß über ihn eine Standardkanüle (14) aufschiebbar ist.

15. Spritze nach einem der Ansprüche 1 bis 14, **dadurch gekennzeichnet,** daß die Spritze mit dem Behältnis (10) für die zu transferierende medizinische Substanz zu einer Lagerungseinheit vormontiert sind.

16. Spritze nach Anspruch 15, **dadurch gekennzeichnet,** daß am Spritzenkorpus (1) in zwei axial beabstandeten Reihen jeweils mindestens zwei wulstartige Ansätze (2 a, b) angeformt sind, die in Wirkeingriff mit einer am Behältnis (10) anbringbaren Halterung (10 a) bringbar sind, wobei der elastomere Verschluß (11) des Behältnisses (10) einen Stutzen (11 a) zur dichtenden Aufnahme zumindest des vorderen Teiles des Hohldornes (9) im Lagerungszustand besitzt.

17. Spritze nach einem der Ansprüche 1 bis 16, **dadurch gekennzeichnet,** daß die Spritze als vorgefüllte Fertigspritze ausgebildet ist.
